# EUROPEAN PATENT APPLICATION

(11) **EP 4 697 347 A1**
(43) Date of publication of application: **18.02.2026**
(21) Application number: 24194372.9
(22) Date of filing: 13.08.2024
(51) Int. Cl.: G16H 30/40, G16H 50/20, G16H 50/30

(54) **DETERMINING RISKS OF HAVING OR DEVELOPING MEDICAL CONDITIONS BASED ON RADIOMICS FEATURES EXTRACTED FROM AUTOMATICALLY SEGMENTED 3D IMAGES OF A SKELETAL MUSCLE OF A SUBJECT'S TRUNK**

(71) Applicant: ECOLE POLYTECHNIQUE FEDERALE DE LAUSANNE (EPFL), 1015 Lausanne (CH)
(72) Inventor: Nachit, Maxime, 1003 Lausanne (CH); El Alam, Gabi, 1004 Lausanne (CH)
(74) Representative: E. Blum & Co. AG

(57) **Abstract**

The invention is notably directed to a computer-implemented method for determining risks of having or developing medical conditions, and/or complications thereof, in a subject. The method comprises: accessing a 3D image of a skeletal muscle of the trunk of the subject, wherein the 3D image has been previously obtained using a medical imaging technique; segmenting (S43 - S44) the 3D image using an automatic image segmentation process; extracting (S45) muscle radiomics features from the segmented 3D image; and executing (S50), based on predetermined sets of weights assigned to respective endpoints, a module to determine a score for each of the endpoints by weighting the extracted muscle radiomics features in accordance with a respective one of the predetermined sets of weights. The score is a risk of having or developing a medical condition or a complication thereof for the respective endpoint, while said medical condition is primarily linked to a system or an organ that is anatomically distinct from said skeletal muscle. The invention is further directed to related devices computerized systems and computer programs.

## Description

### TECHNICAL FIELD

The invention relates in general to the field of a computer-implemented methods, computerized systems, and computer programs for determining risks of having or developing medical conditions, and/or complications thereof, based on radiomics features extracted from an automatically segmented 3D image of a skeletal muscle of the trunk of a subject. In particular, it is directed to methods relying on predetermined sets of weights assigned to respective endpoints, which determine scores of such endpoints by weighting extracted muscle radiomics features in accordance with respective ones of the predetermined sets of weights, where each score is a risk of having or developing a medical condition or a complication thereof for the respective endpoint, and where the corresponding medical condition is primarily linked to a system or an organ that is anatomically distinct from the skeletal muscle of the trunk of the subject.

### BACKGROUND

The term "Radiomics" refers to methods of extraction of features from medical images, which rely on data-characterisation algorithms. The extracted features (the "radiomic features") are used to uncover pathological patterns and other characteristics that may not be detectable by the naked eye.

Radiomics-related research focuses on oncology. Typically, these studies revolve around extracting radiomics from a given organ (or a given tumorous lesion within said organ) to assess a risk of disease related to this organ. For example, radiomics extracted from liver images are used to predict liver diseases. Similarly, brain radiomics are used to investigate neurological conditions, and so on. While radiomics has provided outstanding results, the fact remains that it requires images of each of the organs studied. Conventional radiomics method are therefore inapplicable in the absence of images of the organ concerned.

### SUMMARY

According to a first aspect, the invention is embodied as a computer-implemented method for determining risks of having or developing medical conditions, and/or complications thereof, in a subject. The method first comprises accessing a 3D image of a skeletal muscle of the trunk of the subject, where the 3D image has been previously obtained using a medical imaging technique. Next, the method comprises segmenting the 3D image using an automatic image segmentation process and extracting muscle radiomics features from the segmented 3D image. Finally, the method comprises executing a module, based on predetermined sets of weights assigned to respective endpoints, to determine a score for each of the endpoints by weighting the extracted muscle radiomics features in accordance with a respective one of the predetermined sets of weights. This score is a risk of having or developing a medical condition or a complication thereof for the respective endpoint. That is, each of the endpoints corresponds to a respective medical condition or a complication thereof. Interestingly, this medical condition is primarily linked to a system or an organ that is anatomically distinct from said skeletal muscle. So, the medical conditions associated with the endpoints concern secondary organs, i.e., organs at distance from the reference organ, i.e., the skeletal muscle of the trunk (also called torso) of the subject.

The proposed method leverages the predictive power of radiomics extracted from a skeletal muscle. As the present inventors realized, muscle radiomics features extracted from a skeletal muscle of the trunk (e.g., a paravertebral skeletal muscle) can advantageously be exploited to successfully determine risks related to distinct organs, provided that the relevant muscle radiomics features are identified and suitably weighted first. This approach differs from usual approaches, which use radiomics from one organ to predict a risk for that same organ, or which attempt to find a same subset of relevant radiomics for several endpoints.

The proposed method can adequately predict risks related to several medical conditions, even in the absence of 3D images of the corresponding organs. On the contrary, 3D images (e.g., MRI images) of trunk-level skeletal muscles are often available. Moreover, the proposed weighting scheme is easily scalable as the underlying vectors (or arrays) can simply be concatenated to further take into account additional information, whether radiomics extracted from additional 3D images, genetic information, or medical information.

In embodiments, at least some of the extracted muscle radiomics features include features related to: (i) a pattern of fat infiltration in said skeletal muscle, where said pattern preferably captures one or more textural features of said skeletal muscle, and (ii) a shape of said skeletal muscle or a portion thereof. The inventors observed that associating the spatial pattern of fat infiltration with structural characteristics of the muscle amounts to identifying hidden key variables of the problem, which substantially improve the prediction accuracy.

The degree of fat infiltration (i.e., the quantity of fat in a given muscle volume, which can be expressed as a percentage) may advantageously be added to the spatial pattern of fat infiltration and the structural characteristics of the muscle. Thus, in embodiments, one of the extracted muscle radiomics features further relates to a degree of fat infiltration in said skeletal muscle.

Better results can be obtained by extracting muscle radiomics features that include each of the above features, i.e., (i) the degree of fat infiltration in the skeletal muscle, which preferably is a muscle at the abdominal level of the subject, (ii) the pattern of fat infiltration in the skeletal muscle, where this pattern captures several textural features of the skeletal muscle, and (iii) the shape of the skeletal muscle, or a portion thereof. Beyond the fat infiltration degree, the spatial patterning of fat infiltration and the structural characteristics of muscles turn out to offer valuable clinical perspectives, in combination, especially when taking several textural features into consideration at a time.

In embodiments, the automatic image segmentation process causes to segment the 3D image (e.g., an MRI image) by: obtaining a segmentation mask from a first image obtained from the 3D image; and applying the segmentation mask obtained to a second image obtained from the 3D image to obtain the segmented 3D image. The first image and the second image contain different information in terms of fat content and/or water content. Preferably, the 3D image is an MRI image, the first image is a water map, and the second image is a fat fraction map, whereby the segmented 3D image is obtained as a segmented fat fraction map. Compared with usual automatic segmentation process, such an approach was found to result in more reliable segmentations, which also benefit the subsequent feature extraction and, thus, the eventual risk predictions.

Of note, a similar principle can be applied to CT-scan images. However, in this case, the segmentation mask is typically extracted from the same image to which it is subsequently applied (i.e., there is no water map or fat fraction map, there is a unique map, in which each voxel value is in grey or Hounsfield unit). In principle, however, any medical image (including CT-scan images) may be subject to different types of processing (e.g., filter transformations) to emphasize water and/or fat content, whereby different versions of that image can be obtained to first obtain a segmentation mask and then apply this mask.

The reliability of the segmentation masks obtained can be further improved by using a trained neural network. Namely, in embodiments, the segmentation mask is obtained by running a trained neural network, preferably a convolutional neural network, on the first image (e.g., a water map, should MRI images be used). This neural network is a computational model that is assumed to have already been trained, during a training phase, to infer segmentation masks from a training set of annotated images (e.g., annotated water maps).

The inventors observed strong associations between radiomics and the likelihood of adverse health outcomes with unexpected diseases, which are not obviously linked to skeletal muscles, such as Parkinson's disease, acute pancreatitis, and renal diseases. Thus, in embodiments, the endpoints are associated with medical conditions that include Parkinson's disease, acute pancreatitis, and/or a renal disease (e.g., chronic, and/or acute renal failures). The endpoints are preferably associated with medical conditions that include each of the above diseases. More generally, the present methods can adequately predict the risk of several diseases, further including various liver diseases, heart failure, chronic ischemic heart diseases, myocardial infarction, stroke, type II diabetes, and all-cause mortality.

The weighting scheme is easily scalable as the underlying vectors can simply be concatenated. Namely, in embodiments, the extracted muscle radiomics features are weighted in accordance with said respective one of the predetermined sets of weights by performing, for each of the endpoints, a dot product of two vectors. The first vector includes the extracted muscle radiomics features, while the second vector includes said one of the predetermined sets of weights (i.e., the weights assigned to the types of muscle radiomics features for the endpoint considered).

Now, the method may further comprise, prior to performing said dot product, concatenating additional data (i.e., vector components) with each of the two initial vectors corresponding to the muscle radiomics features extracted, in order to account for additional information (e.g., one or more additional 3D images, genetic information, medical information), should additional information be available and used, as in embodiments. I.e., on the one hand, additional features can be concatenated with the extracted muscle radiomics to form the first vector (the additional features reflect the additional information). On the other hand, additional weights (as assigned to these additional features) can be concatenated with said one of the predetermined sets of weights (i.e., the weights assigned to the sole muscle radiomics features for a respective endpoint). This way, the method can easily take additional information into account to refine the risk predictions.

For instance, in embodiments, the module further takes patient information and/or genetic information of the patient as input, in addition to the muscle radiomics features. This genetic information preferably contains single nucleotide polymorphisms (SNPs), more preferably myosteatosis-associated SNPs. Any useful additional information can easily be taken into consideration by concatenating corresponding vectors (or arrays). Similarly, the predetermined sets of weights can include additional weights corresponding to such additional information.

Since the present approach is easily scalable, radiomics of additional 3D images may advantageously be added, should they be available. For instance, in embodiments, the method further comprises accessing an additional 3D image of an additional organ that is anatomically distinct from the reference organ (skeletal muscle), where this additional 3D image has previously been obtained using a medical imaging technique. The additional 3D image is again segmented using an automatic image segmentation process and additional radiomics features are extracted from the segmented, additional 3D image, to obtain a set of radiomics features that include both the muscle radiomics features and the additional radiomics features. The module is executed to determine said score for each of the endpoints by weighting the radiomics features of this set. Note, one or more of the medical conditions associated with the endpoints may primarily be related to this additional organ.

In embodiments, the method further comprises performing a preliminary analysis, which comprises accessing a set of 3D images of skeletal muscles of trunks of multiple subjects. The 3D images have been previously obtained using said medical imaging technique, e.g., magnetic resonance imaging. The set of 3D images preferably includes at least 30000 3D images of respective subjects and, preferably at least 40000 3D images. Next, the 3D images are segmented using said automatic image segmentation process. Sets of muscle radiomics features are then extracted from the segmented 3D images and a dimension reduction is performed on all of the sets of extracted radiomics features to obtain linear combinations of the radiomics feature values extracted. Not only this dimension reduction lowers the risk of overfitting but, in addition, it subsequently permits to substantiate the clinical relevance of the radiomics. That is, the linear combinations are subsequently mapped, statistically, onto the endpoints, while the sets of (relevant) weights are determined thanks to constants used in the linear combinations. The sets of weights determined are then assigned to their respective endpoints.

Interestingly, the required sets of weights can be determined thanks to the constants that appear in the linear combinations; such constants are always the same for each of the sets of extracted features. Eventually, the determined sets of weights can be assigned to their respective endpoints.

In principle, several linear reduction methods can be contemplated. However, the dimension reduction is preferably performed using a principal component analysis (PCA), by keeping only the first L principal components, where L is preferably between 8 and 12, and more preferably equal to 10. This approach guarantees that the linear combinations that are the most relevant, statistically (i.e., the first L principal components), are considered. In addition, a PCA analysis is fairly simple and robust, and also tends to minimize the information loss.

In embodiments, the linear combinations are statistically mapped onto the endpoints by running a time-to-event model, which preferably is a Cox proportional hazards time-to-event model. Accordingly, the subsequent step of weighting the extracted muscle radiomics features (as executed at runtime) results in time-dependent quantities, which are preferably expressed as relative risk measures (or hazard ratios). This makes it possible to predict risks over time.

According to another aspect, the invention is embodied as a computerized system for determining risks of having or developing medical conditions, and/or complications thereof, in a subject. The computerized system comprises an interface unit and processing means. The interface unit is configured to receive 3D images that have been previously obtained using a medical imaging technique. The processing means are configured to perform steps as described above, i.e., access a 3D image of a skeletal muscle of the trunk of the subject, segment the 3D image using an automatic image segmentation process, extract muscle radiomics features from the segmented 3D image, and execute a module, based on predetermined sets of weights assigned to respective endpoints, to determine a score for each of the endpoints by weighting the extracted muscle radiomics features in accordance with a respective one of the predetermined sets of weights. Again, said score is a risk of having or developing a medical condition or a complication thereof for the respective endpoint, and said medical condition is primarily linked to a system or an organ that is anatomically distinct from said skeletal muscle.

A final aspect concerns a computer program comprising software code adapted to perform a method for determining risks of having or developing medical conditions, and/or complications thereof, in a subject, according to any of the embodiments described above in reference to the first aspect of the invention.

### BRIEF DESCRIPTION OF THE DRAWINGS

These and other objects, features and advantages of the present invention will become apparent from the following detailed description of illustrative embodiments thereof, which is to be read in connection with the accompanying drawings. The illustrations are for clarity in facilitating one skilled in the art in understanding the invention in conjunction with the detailed description. In the drawings:
FIG. 1 schematically represents a general-purpose computerized system, suited for implementing one or more method steps as involved in embodiments of the invention;
FIG. 2 is a flowchart illustrating steps of a preliminary analysis to identify clinically relevant radiomics features, and weights thereof, for several endpoints (corresponding to medical conditions or complications thereof), as involved in embodiments;
FIG. 3 is a flowchart illustrating high-level steps of a method for determining risks of having or developing medical conditions, and/or complications thereof, in a subject, based on radiomics features extracted from a skeletal muscle of a trunk of a subject, according to embodiments;
FIG. 4 illustrates an automatic image segmentation process of 3D images obtained by magnetic resonance imaging, as involved in embodiments. The images shown in FIG. 4 have been intentionally reworked for depiction purposes. Such images markedly differ from real images as processed by the present methods; and
FIG. 5 shows diagrams illustrating how weights of relevant radiomics features can be mapped onto endpoints, as involved in embodiments.

The accompanying drawings show simplified representations of devices or parts thereof, as involved in embodiments. Similar (or functionally similar) elements in the figures have been allocated the same numeral references, unless otherwise indicated.

Computerized systems, methods, and computer program products embodying the present invention will now be described, by way of non-limiting examples.

### DETAILED DESCRIPTION OF EMBODIMENTS OF THE INVENTION

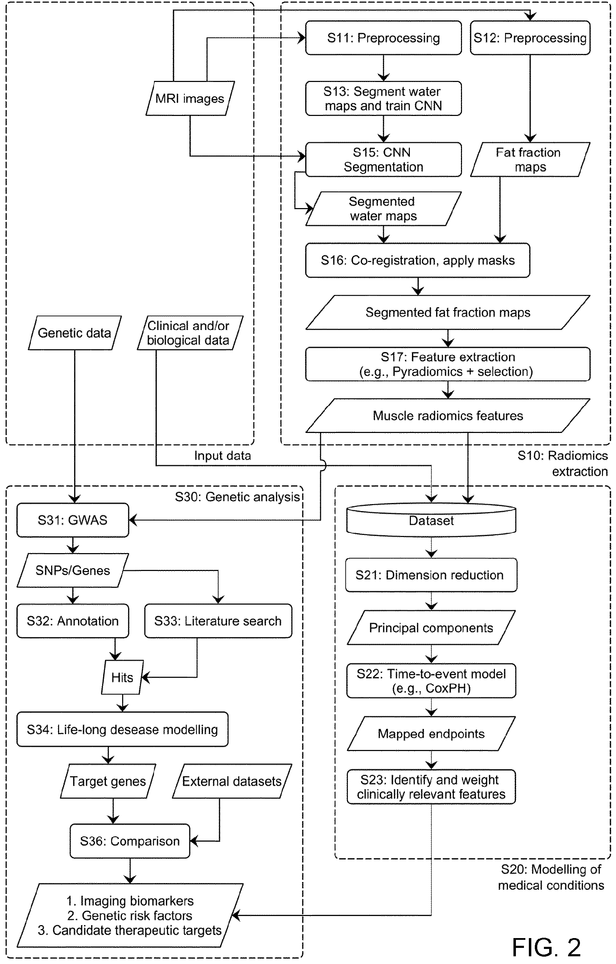

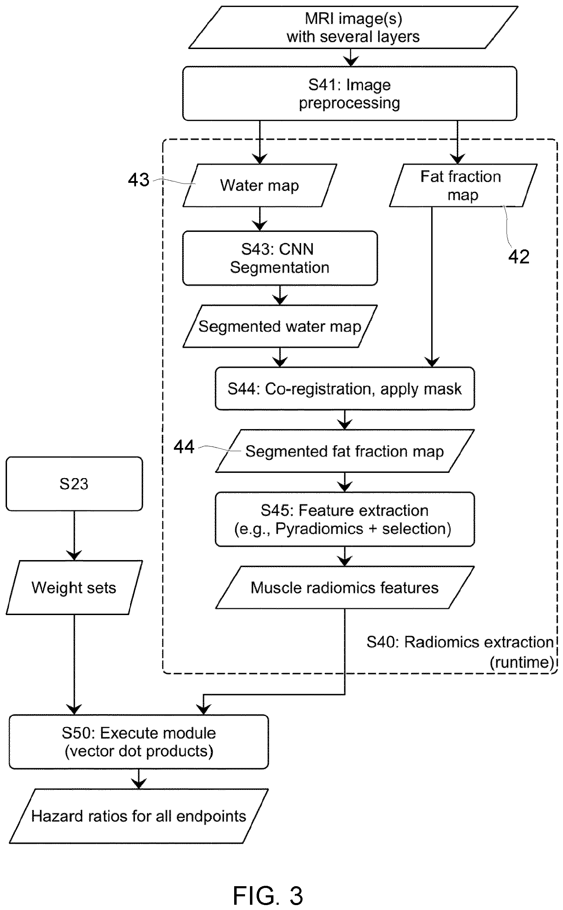

The following description is structured as follows. General embodiments and high-level variants are described in section 1. Section 2 addresses particularly preferred embodiments. Section 3 concerns technical implementation details. Note, the present method and its variants are collectively referred to as the "present methods." All references Sn refer to methods steps of the flowcharts of FIGS. 2 and 3, while numeral references pertain to devices, components, and other concepts as involved in embodiments of the present invention.

### 1. General embodiments and high-level variants

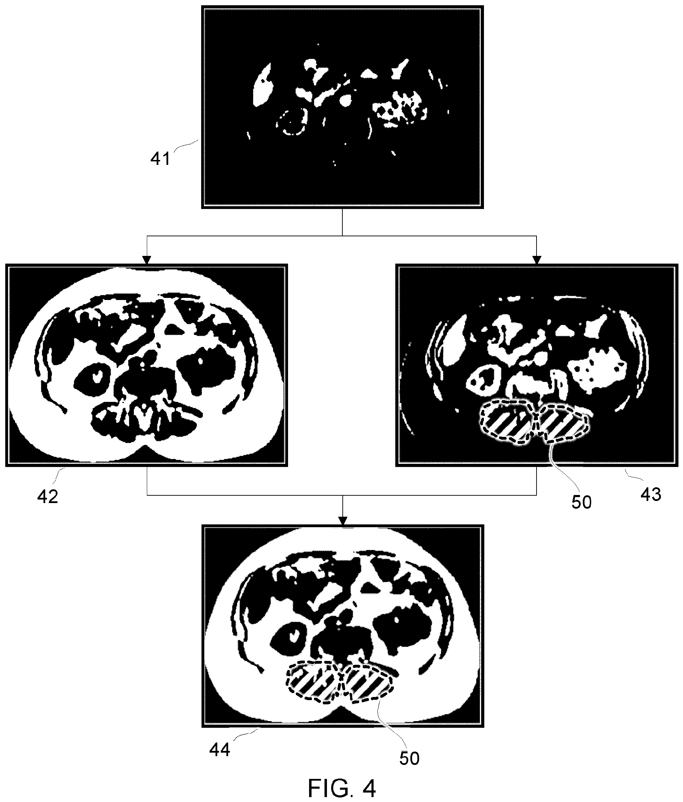

Referring to FIGS. 3 and 4, a first aspect of the invention concerns a computer-implemented method for determining risks of having or developing medical conditions, and/or complications thereof, in a subject (e.g., a human or animal).

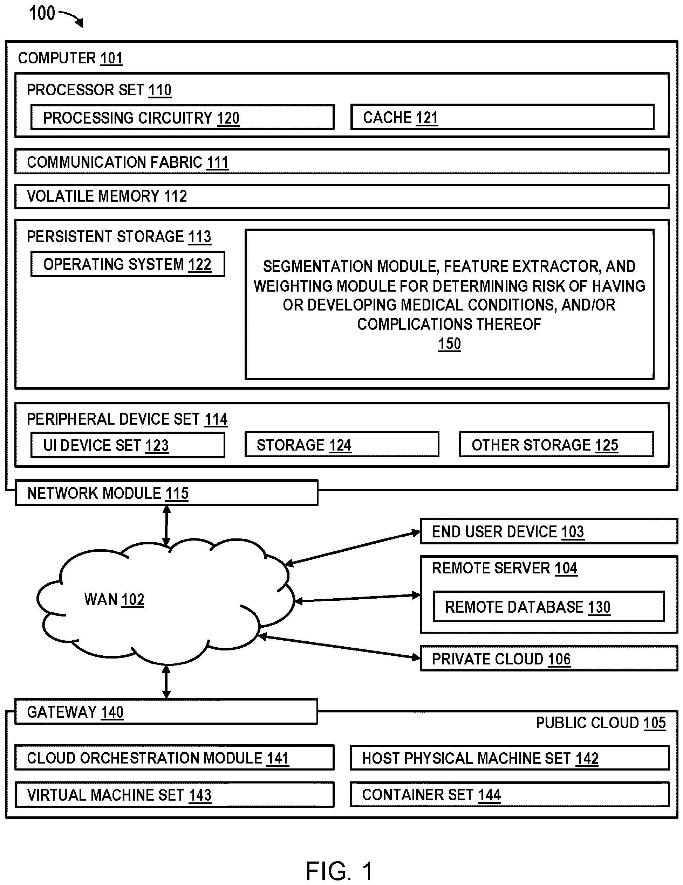

The method relies on 3D images, which are obtained thanks to a medical imaging technique, such as magnetic resonance imaging (MRI), computed tomography (CT) scan, or ultrasound (US) imaging. However, the method preferably uses MRI or CT scan images, given that radiomics are less reproducible on US imaging. More preferably, use is made of MRI images, which allow a better assessment of fat infiltration. In all cases, such images are assumed to be already available at the time of performing the present methods. These methods are typically implemented on a computer environment such as shown in FIG. 1. This, however, concerns another aspect of the invention, which is described later in detail.

The method first accesses a 3D image 41 of a skeletal muscle. The 3D image may be an image of only a portion of the skeletal muscle, also referred to as the "reference organ" herein. This skeletal muscle is a muscle of the trunk (or torso) of the subject, i.e., the upper body for a human, excluding limbs and the head. This muscle is preferably a muscle at the abdominal level. For example, this muscle may be the rectus abdominis, internal or external abdominal oblique, cremaster, transversus abdominis, pyramidalis, or quadratus lumborum muscle, and also the paraspinal muscles (i.e., erector spinae and multifidus), also known as paravertebral muscle, amongst other examples.

Next, the 3D image 41 is segmented (see steps S43 - S44 in the flow of FIG. 3), using an automatic image segmentation process. The automatic image segmentation process may notably involve a trained neural network, e.g., a convolutional neural network (CNN), as well as other procedures, such as image registration procedures. This neural network is a computational model that has already been trained to infer segmentation masks from a training set of annotated water maps, as further discussed later.

Muscle radiomics features (MRFs) are then extracted (step S45) from the segmented 3D image 44. The MRFs are typically extracted as a vector (also called array) of scalar values (i.e., vector components), which may be subject to various procedures, e.g., rescaling, normalization. Use can for instance be made of the so-called Pyradiomics pipeline. Invariant features (e.g., features that are identical across all subjects) are preferably removed. Alternatively, they may be assigned a zero weight.

Finally, a module is executed S50 based on predetermined sets of weights that have previously been assigned to respective endpoints. These endpoints are associated with respective medical conditions or complications thereof. The endpoints may notably include Parkinson's disease, Pancreatitis, and/or renal diseases, as well as potential complications of such diseases. More generally, the present methods can adequately predict risks related to several diseases, also including various liver diseases, heart failure, chronic ischemic heart diseases, myocardial infarction, stroke, type II diabetes, and all-cause mortality. Such medical conditions preferably exclude cancer diseases, for which other various methods are available. That is, the selected endpoints are preferably not associated with conditions/complications that are primarily related to cancer.

The module is an executable set of instructions, which can possibly restrict to a mere callable unit, i.e., a function, method, operation, subroutine, procedure, etc., that is, any structural unit that can be executed to produce or alter data. Executing this module causes to determine a score for each of the endpoints, by weighting the extracted MRFs in accordance with a respective one of the predetermined sets of weights (a different weight set is used for each endpoint). That is, the module executes based on the predetermined sets of weights, which have been assigned to respective endpoints.

The obtained score can be interpreted as a risk of having or developing a medical condition or a complication thereof for the respective endpoint. Importantly, this medical condition is primarily linked to a system or an organ that is anatomically distinct from the reference organ, i.e., the skeletal muscle. The same holds for each endpoint and, thus, each corresponding medical condition or complication.

In other words, the endpoints correspond to medical conditions/complications that are primarily related to secondary organs (also referred to as "at-distance organs" or target organs herein), i.e., organs that differ from the reference organ (a skeletal muscle of the trunk). So, in the present context, the medical conditions (or health conditions) and/or complications associated with the endpoints include diseases and other physiological disorders, which primarily relate to organs (e.g., heart, kidneys, and lungs) that differ from the reference organ. Several endpoints are considered (not just one), which may typically include dozens to hundreds to thousands of endpoints notably when considering complications, in addition to medical conditions.

Comments are in order. While the above method refers to a skeletal muscle and a 3D image 41 thereof, it should be noted that this method can be applied to several 3D images of the same muscle (or portions thereof) or even to several 3D images of different skeletal muscles (or portions thereof) of the trunk of the subject. That is, the reference organ(s) considered herein may actually include one or more skeletal muscles (or portions thereof) and use can be made of one or more 3D images of such muscles.

More generally, additional organs may possibly be considered, in addition to the skeletal muscle evoked above. And such additional organs may possibly concern medical conditions associated with the endpoints looked for. However, even in that case, the medical conditions associated with the endpoints primarily relate to a system or an organ that is anatomically distinct from the reference skeletal muscle(s).

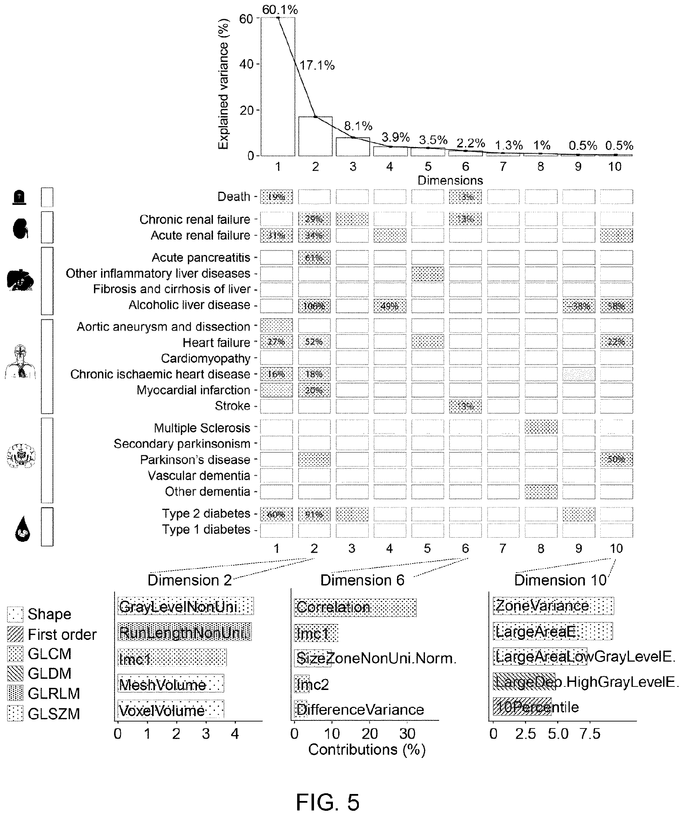

The weights used in the weighting scheme capture key information; they can be initially determined according to procedures such as described later in reference to FIGS. 2 and 5. Since the radiomics features can normally be expressed as vectors, weighting the radiomics features amounts to performing a dot product of two vectors, which respectively capture the weights assigned to a given endpoint and the extracted feature values. In operation, the module may for instance look up a mapping table, which identifies a set of weights for each endpoint, and then perform a dot product for each endpoint. Dot products are linear operations, which are easily and quickly performed. For this reason, the present methods are easily scalable to multiple images and weight sets, as discussed later in reference to particular embodiments.

Instead of vectors, the extracted features values may possibly be captured as matrices. In that case, the weight sets may be captured as matrices, too, hence giving rise to matrix multiplications, which remain linear operations. The outcome of such matrix multiplications, however, would typically have to be compressed to a single (scalar) value. In all cases, the output of the weighting scheme is formulated as a score, which can be interpreted as a risk of having or developing a medical condition or a complication thereof for each of the respective endpoints, possibly a risk over time.

This weighting scheme amounts to establishing associations between MRFs and respective endpoints. Such associations are explicit (each weight is associated to a given MRF), as opposed to associations that could be implicitly captured in hidden layers of a trained machine learning model, for example. As a result, the proposed method is fully explainable, as opposed to usual (black box) machine learning approaches.

As the present inventors realized, MRFs extracted from a skeletal muscle of the trunk can advantageously be exploited to successfully determine risks related to distinct organs, provided that relevant MRFs are identified and suitably weighted first. So, a key aspect of this invention is to leverage knowledge extracted from a reference organ (a trunk-level, skeletal muscle) to determine scores for endpoints related to distinct secondary systems and/or organs.

The above method concerns operations performed at runtime, by an automated system, which processes physiological measurements (in the form of 3D images obtained by a given medical imaging technique), to eventually allow, confirm, or complement a medical diagnosis. It relies on the extraction of distinct radiomics features from one or more skeletal muscles. In its simplest implementation, the method leverages the predictive power of radiomics derived from just one organ (paravertebral skeletal muscle), providing a medical diagnosis by an automated system. However, this implies that a preliminary analysis was performed to obtain the required weights. The latter are coefficients that identify and weight the clinically relevant radiomics for each of the potential endpoints considered. This approach differs from usual approaches, which use radiomics from one organ to predict a risk for that same organ, or which attempt to find a same subset of relevant MRFs for several endpoints. On the contrary, in the present context, distinct subsets of MRFs are identified and weighted for each of several endpoints, by leveraging non-intuitive relationships, which remain largely underexplored to date.

That being said, the module may further take into account additional MRFs, these possibly including MRFs of the target organs, with little additional complexity (e.g., corresponding vectors can just be concatenated). In addition, use can be made of genetic information, as discussed later in detail, in reference to particular embodiments. Beyond the above weighting scheme, which is based on extracted features, the underlying module may further take into account additional data, such as clinical and/or biological data e.g., Post-MRI health record data related to incident diseases as previously obtained for the subject.

All this is now described in detail, in reference to particular embodiments of the invention. To start with, at least some of the extracted MRFs will preferably include features related to a pattern of fat infiltration (i.e., myosteatosis) in the skeletal muscle, which preferably is a muscle at the abdominal level of the subject. This pattern may notably capture one or more textural features of said skeletal muscle. Such textural features (also called texture features) may for instance be computed from one or more matrices generated from the 3D image 41 considered, for instance the so-called Gray Level Co-occurrence Matrix (GLCM), Gray Level Dependence Matrix (GLDM), Gray Level Run Length Matrix (GLRLM), and Gray Level Size Zone Matrix (GLSZM), as referred to in FIG.5. All such features can be generated from the 3D image 41 considered or from one or more transformed versions of this image, e.g., as obtained by applying filters on it. When several textural features are taken into consideration, care should be taken to select features that are not too correlated.

Features related to the pattern of fat infiltration can further be complemented by features relating to the shape of the skeletal muscle (or a portion thereof). Features related to the shape of the skeletal muscle may notably be 3D shapes, e.g., in the form of 3D contours. Still, 2D shapes may additionally be included. Such features reflect structural characteristics of the skeletal muscle. The inventors realized that associating the spatial pattern of fat infiltration with structural characteristics of the muscle amounts to identifying key variables that substantially improve the prediction accuracy.

Moreover, use can be made of one or more MRFs related to a degree of fat infiltration in the skeletal muscle. Such MRFs may notably reflect a fraction, concentration, or some other proportion-related quantity of fat in the muscle. Prior research has characterized myosteatosis based on the average fat concentration within a muscle. However, beyond the mere fat degree (e.g., the mean fat concentration), here the spatial pattern of fat infiltration is taken into consideration, along with the structural characteristics of muscles. The inventors propose that beyond the mean fat concentration, the spatial patterning of fat infiltration and the structural characteristics of muscles offer valuable clinical perspectives, in combination. These attributes, discernible through radiomics, happen to provide a more comprehensive understanding of myosteatosis and its implications on human health.

Use can for instance be made of the so-called Pyradiomics pipeline (https://pyradiomics.readthedocs.io), which makes it possible to extract the following features: First Order Statistics (19 features); Shape-based 3D (16 features); Shape-based 2D (10 features); Gray Level Co-occurrence Matrix (24 features); Gray Level Run Length Matrix (16 features); Gray Level Size Zone Matrix (16 features); Neighbouring Gray Tone Difference Matrix (5 features); and Gray Level Dependence Matrix (14 features). However, some of the features can be discarded, for example the shape-based 2D features. Before extracting MRFs, the 3D images may be subject to various kinds of pre-processing steps, if only to normalize or adjust the images, or filter out undesired components (e.g., noise, if any), for example.

In embodiments, the automatic image segmentation process causes to segment the 3D image 41 according to a process illustrated in FIG. 4, which allows for more faithful segmentations. That is, a fat fraction map 42 of the skeletal muscle is first obtained (see also FIG. 3), along with a water map 43 of the skeletal muscle. In practice, such maps may already be made available by the medical imaging device manufacturer software. In variants, one may prefer to recompute such maps, using different algorithms and/or parameters. Next, a segmentation mask 50 is obtained S43 from the water map 43, and this segmentation mask 50 is then applied (at step S44) to the fat fraction map 42 to obtain the segmented 3D image 44 as a segmented fat fraction map. Note, FIGS. 3 and 4 assume the use of MRI images. In that case, the fat fraction map 42 and the water map 43 can be regarded as distinct layers of the same 3D image 41. Because different images 42, 43 are used, the image containing the segmentation mask extracted may have to be co-registered with the fat fraction map, prior to applying the segmentation mask at step S44.

More generally, use can be made of different images 42, 43 (or different versions of a same image), which capture different information in terms of water content and/or fat content. Note, there is no need to co-register images when using different versions of a same image, prior to applying the segmentation mask extracted. In particular, when using CT scan images, the very same image could be used to extract the segmentation mask and then apply this mask, hence eliminating the need to co-register images.

Using the Pyradiomics pipeline, one may for instance extract 100 skeletal muscle radiomics features from the segmented fat fraction image. One will preferably exclude invariant features (e.g., "Min," "Max," and "Range"), which results in 97 useful radiomics features. In embodiments, the segmentation mask is obtained S43 by running a previously trained neural network (preferably a CNN), on the water map 43. I.e., the neural network takes voxel values of the water map as inputs. More generally, such a neural network may be run on a first image 43, to extract a segmentation mask that is then applied to a second image 42, as explained above. Initially, the neural network can be trained on annotated images (e.g., water maps), as further discussed in section 2.

In embodiments, the module further takes patient information and/or genetic information of the patient as input, in addition to the MRFs. This genetic information preferably contains single nucleotide polymorphisms (SNPs) and, more preferably, myosteatosis-associated SNPs. The genetic analysis aims at obtaining a depiction of the genetic risk factors. Genetic risk factors can be used together with clinically relevant radiomics to determine the endpoints more accurately. This additional information can again be weighted, based on predetermined weights. In practice, genetic information can be used to complement the vector (or array) of extracted MRF values. In other words, MRFs are augmented with additional information (e.g., SNPs), to make better prediction when inferring endpoint scores. In addition, this additional information may further be used to infer therapeutic suggestions, as suggested in the flow of FIG. 2. The relevant genetic information can be determined thanks to a genome-wide association study GWAS on a cohort of individuals, as further discussed in section 2.

Further information may possibly be taken into account. For instance, in embodiments, use is made of 3D images 41 (e.g., MRI images) of additional organs that are anatomically distinct from the refence skeletal muscle. Again, such images are segmented S43 - S44 and additional radiomics features are extracted S45 from the segmented images, following a similar pipeline as described above in reference to FIG. 4. This way, a superset of the MRFs is obtained. This set includes both the MRFs and the additional radiomics features. Eventually, the module is executed S50 to determine a score for each of the endpoints by suitably weighting all radiomics features of the set of features obtained. Note, unlike the reference skeletal muscle, the additional organs considered here may possibly relate (directly) to the medical conditions associated with the endpoints. That is, one or more of such medical conditions may primarily be related to one or more of the additional organs taken into consideration.

Referring now to FIG. 2, the present methods typically follow a preliminary analysis S10 - S30, which relies on a set of 3D images of skeletal muscles of trunks of multiple subjects. Such 3D images must have been obtained using the same medical imaging technique as used at runtime, for example MRI. The set of 3D images considered should be fairly large, for statistical reasons. In the present context, the present inventors came to conclude that such a set should preferably include at least 30000 images of (trunks of) respective subjects, at more preferably at least 40000 images. The reason for doing so is that only a small percentage of the subjects may have medical conditions of interest. As such medical conditions are normally rare in the general population (in a short timeframe, each medical condition typically affects less than 1% of the population), considering a large number of subject images typically gives rise to at least a few hundred images for each medical condition of interest, hence allowing adequate statistics. However, the set of images can be reduced when focusing on unhealthy subjects.

The 3D images are segmented S13 - S16 using an automatic image segmentation process similar to that used at runtime. Before doing so, a neural network model can be trained S13 to extract segmentation masks from suitably annotated water maps (e.g., obtained from a subset of the input images or another training set), with a view to applying S16 such masks to fat fraction maps 42 obtained from the input images, as done at runtime.

Next, sets of radiomics features are extracted S17 from the segmented 3D images (one set of MRFs is extracted from each image). A dimension reduction is subsequently performed S21 based on all sets of extracted radiomics features to obtain linear combinations of the radiomics feature values extracted. That is, the dimension reduction function learns the relevant linear combinations. This step lowers the risk of overfitting and, in addition, it permits to substantiate the clinical relevance of the radiomics during the next step. That is, the linear combinations obtained are statistically mapped S22 onto the endpoints, using knowledge available for the cohort of subjects considered.

The beauty of this approach is that the required sets of weights can still be determined S23, thanks to the constants that appear in the linear combinations; such constants are always the same for each of the sets of extracted features. Eventually, the determined sets of weights can be assigned to their respective endpoints. From this point on, all required ingredients are ready for use in the runtime process (FIG. 3). That said, other approaches may potentially be contemplated to obtain suitable weights. In particular, such weights could be systematically learned, using cognitive techniques. Still, the above approach is believed to be more accurate.

In principle, several linear reduction methods can be contemplated. However, the dimension reduction is preferably performed using a principal component analysis (PCA), by keeping only the first L principal components, where L is preferably between 8 and 12, and more preferably equal to 10. Not all the principal components need be kept. Rather, one preferably keeps only the first L principal components by using only the first L eigenvectors, i.e., the eigenvectors that correspond to the largest eigenvalues. This approach guarantees that the statistically most relevant linear combinations (i.e., the L first principal components) are considered. Other dimension reduction methods could possibly be contemplated, e.g., based on other factor analysis model, such as the so-called exploratory factor analysis. However, a PCA analysis is fairly simple and robust, and also tends to minimize the information loss.

The sets of linear combinations are statistically mapped onto the endpoints at step S22. In embodiments, this steps involves a time-to-event model, such as a Cox proportional hazards time-to-event model. This way, the subsequent weighting scheme (as performed at runtime) results in time-dependent quantities, which can for instance be expressed as relative risk measures (also called hazard ratios). Running a time-to-event model effectively amounts to performing a time-to-event analysis. Beside the CoxPH model, other time-to-event models may possibly be contemplated, such as the so-called Fine Gray model and Caplan-Meyer model.

For example, a PCA model can be used for the dimension reduction. One can then extract individual feature contributions from the linear combinations. Preferably, only the first 10 principal components are kept (call them PC1 to PC10). Next, a linear regression model (e.g., a CoxPH model) may reveal that, for example, PC10 is fairly highly associated with Parkinson's disease, as is the case in FIG. 5. In addition, the constants obtained from the PCA tells how much each feature contributes. From this, one understands that it is possible to reconstruct weight sets for each endpoint. Next, the weighting scheme used at runtime amounts to characterize the role played by subsets of the most relevant features for each endpoint. This results in a fully explainable model, which outputs a risk of having or developing given health states or medical conditions.

As noted earlier, one will preferably remove invariant features (i.e., features that are invariant across subjects) from the extracted features. In that case, the feature values fed into the module are free of invariant features. In practice, the feature extractor may for instance extract a set of 100 radiomics features from each input image. After removing the uninformative, invariant features, 97 features remain, which advantageously include features related to myosteatosis and muscle shapes. Additional details are provided in section 2.

Referring to FIG. 1, another aspect of the invention is now discussed, which concerns a computerized system 100 for determining risks of having or developing medical conditions, and/or complications thereof, in a subject. Functional aspects of such a system have already been described, if only implicitly, in reference to the present methods. Accordingly, the system is only briefly described in the following.

The system may for instance restrict to a mere computer 101, or be a whole computing environment 100, the general architecture of which is shown in FIG. 1. The computerized system comprises one or more interface units 114, 115, which are configured to receive 3D images that have been previously obtained using a medical imaging technique. It further includes processing means 110, 112, 150, which are configured to access a 3D image 41 of a skeletal muscle of the trunk of the subject, segment the 3D image 41 using an automatic image segmentation process as described earlier, and extract MRFs from the segmented 3D image 44.

In addition, the processing means are configured to execute a module (or any callable unit), based on predetermined sets of weights assigned to respective endpoints, to determine a score for each of the endpoints. In operation, module causes to weight the extracted MRFs in accordance with a respective one of the predetermined sets of weights, i.e., one weight set of each endpoint. Again, this score is a risk of having or developing a medical condition or a complication thereof for each of the respective endpoints, and the corresponding medical condition is primarily linked to a system or an organ that is anatomically distinct from said skeletal muscle. This aspect is further described in section 3.

A final aspect concerns a computer program comprising software code adapted to perform a method for determining risks of having or developing medical conditions, and/or complications thereof, in a subject, according to any of the embodiments described above in reference to the first aspect of the invention. The computer program may also be embodied as a computer program product, as discussed in detail in section 3.

The above embodiments have been succinctly described in reference to the accompanying drawings and may accommodate a number of variants. Several combinations of the above features may be contemplated. Examples are given in the next section.

### 2. Particularly preferred embodiments

Myosteatosis is increasingly recognized as a significant health concern, independently from obesity.

The following (section 2.1) reports an analysis of MRI data from 43077 participants in the UK Biobank, using deep-learning to 3D-segment the paravertebral skeletal muscles and extract radiomics features related to lipid content, infiltration patterns, and muscle shape. Muscle radiomics were strongly linked to higher mortality risks and the development of various diseases, including Parkinson's, chronic renal, cardiac, and liver diseases, acute pancreatitis, and type 2 diabetes. Through a GWAS study, the inventors unveiled genetic underpinnings of myosteatosis, identifying single nucleotide polymorphisms (SNPs) associations within 56 genes. They explored their impact on lifelong disease risk by analysing data from 372123 individuals who underwent whole exome sequencing and by subsequent cross-comparison with FinnGen and the Open Target platform. The radiogenomic pipeline they used reveals intricate relationships between skeletal muscle health, its genetic determinants, and lifelong diseases risks, providing a novel perspective for personalized health management.

Practical application (section 2.2) is made to the prediction of risks of having or developing medical conditions, and/or complications thereof, in a subject, using an MRI image of a skeletal muscle of the trunk of the subject.

### 2.1. Preliminary analysis, FIG. 2

Extending previous research, the inventors came to the conclusion that myosteatosis is not only associated with many diseases susceptibilities but likely have genetic underpinnings in humans. While prior research has typically characterized myosteatosis based on the average fat concentration within a muscle, here the inventors propose that, beyond the mean fat concentration, the spatial patterning of fat infiltration and the structural characteristics of muscles offer valuable clinical perspectives. These attributes, discernible through radiomics, turn out to provide a more comprehensive understanding of myosteatosis and its implications on human health.

In particular, the inventors found strong associations between skeletal muscle radiomics and the likelihood of adverse health outcomes. This ranges from type 2 diabetes, liver diseases, and overall mortality, to unexpected diseases, not obviously linked to skeletal muscles, such as renal conditions, acute pancreatitis, heart conditions, and Parkinson's disease.

Next, leveraging radiomics, the inventors have explored the genetic architecture underlying skeletal muscle shape and lipid distribution using GWAS. In doing so, the inventors pinpointed over a hundred SNPs within genes that have a clear link to skeletal muscle biology, such as ACVR2B, CACNA1S, and SPEG. Additionally, they identified numerous other SNPs that, to date, show little or no obvious relation to these muscle traits. To explore their potential clinical relevance, the inventors evaluated the influence of muscle-associated SNPs on the lifetime risk of developing diverse diseases. Their analysis revealed potent associations with chronic conditions of the liver, heart, and kidneys, T2D, as well as neurodegenerative diseases, such as Parkinson's disease, Alzheimer's disease, and multiple sclerosis. The analysis flow used by the inventors is discussed in the next section.

### 2.2. Cohort analysis, FIG. 2

Input data include a set of more than 43077 whole-body MRI images, clinical data (e.g., age, sex, BMI, smoking habits) as well as genetic data and/or biological data. The participants were scanned in a Siemens MAGNETOM Aera 1.5 T MRI scanner (Siemens Healthineers, Erlangen, Germany) using a 6 min dual echo Dixon Vibe protocol, providing a water and fat separated volumetric data set covering neck to knees.

Step S10 generally refers to radiomics extraction. The input MRI images are typically subjected to various preprocessing steps S11, S12, if only to normalize (or otherwise adjust) the images, and/or filter out undesired image components. Distinct preprocessing may be used for the two pipeline paths (respectively under steps S11 and S12). One of these paths concerns the segmentation of water maps. A subset of the input MRI images may be used to train a CNN (e.g., based on pre-segmented images, S13), for it to learn S13 to extract suitable segmentation masks from the input images. Once trained, the CNN is used to obtain S15 segmentation masks for the remaining water maps. The other path concerns the fat fraction maps, which are generated using the so-called RECOH pipeline (https://github.com/recoh/pipeline/). For each input image, the corresponding skeletal muscle mask is co-registered with the fat-fraction map at step S16, and the segmentation mask inferred S15 for the water map is used to segment the fat fraction map. Segmented fat fraction maps are accordingly obtained, from which MRFs are extracted, e.g., using the Pyradiomics pipeline. A selection of MRFs is performed, whereby invariant features are discarded, as well as features related to 2D shapes. The remaining features essentially concern 3D shapes, fat infiltrations patterns, and the fat content. The residual muscle radiomics features are temporarily stored in the main memory of the system 100 for further processing, step S20. Alternatively, or in addition, such data can be added to a dataset, in addition to other input data, such as clinical and/or biological data.

Step S20 generally concerns the modelling of medical conditions. At step S21, the extracted MRFs are subject to a dimension reduction based on PCA, yielding a number of principal components (PCs). Only the first 10 PCs are kept, which are fed to a time-to-event model (e.g., CoxPH), for it to map S22 the PCs onto the selected endpoints. Finally, the clinically relevant features are identified and weighted at step S23, by exploiting constants (i.e., static weights) of the PCs.

The genetic analysis S30 can be performed at any time, e.g., before, after, or in parallel with, the modelling step S20. This analysis uses the available genetic information, in addition to the extracted MRFs. A GWAS study is first performed S31 on the same cohort as used to extract the MRFs. Namely, the GWAS study is performed on the MRFs, not only to map their underlying genetic architecture, but also to assess whether these features associate with the risk of future disease development. The impact of muscle radiomics-derived SNPs (or genes) on lifelong disease risk is evaluated on a distinct set of participants, i.e., distinct from the discovery cohort with MRI, and with available whole-exome sequencing data. The output is compared S36 to pre-existing knowledge from external datasets (Open Target Platform and FinnGen). This preliminary analysis made it possible to identify clinically relevant genetic associations and their respective weights across life-long incidence of several endpoints, through a life-long disease modelling S34. The analysis includes annotating S32 the identified genetic variants using current knowledge from literature and annotation databases, step S33. This, together with the clinically relevant features identified S23, made it possible to identify imaging biomarkers, genetic risk factors, and candidate therapeutic targets.

Knowledge gained from the above study S10 - S30 is then exploited to perform real-time predictions from even a single MRI image of a skeletal muscle of the trunk of a given subject, as discussed in the next section.

### 2.2. Preferred runtime process flow, FIG. 3

Step S40 generally refers to the radiomics extraction process used at runtime. MRI image(s) are again used as input; such image(s) now relate to one subject at a time, although several images could possibly be processed in parallel. The runtime process is typically meant to perform predictions for previously unseen subject's images. Such images may again be pre-processed, possibly differently for each of the two pipeline paths discussed below. The preset algorithms of the MRI tool are used to compute the water map and the fat fraction map. The trained CNN is used to segment S43 the water map and obtain a segmented water map. The segmentation mask obtained is then applied S44 to segment the fat fraction map, after co-registering the mask image with the fat fraction map. A segmented fat fraction map is accordingly obtained. At step S45, a feature extraction is performed, e.g., using Pyradiomics, and invariants are preferably discarded, which leads to 97 muscle radiomics features (for each image considered). A module is then executed at step S50, which basically performed a vector dot products of each weight array obtained from step S23 and the extracted MRFs, leading to the desired hazard ratios for all the endpoints considered.

### 2.4. Identifying and weighting muscle radiomics in predictive modelling of incident adverse events, FIG. 5

The top part of FIG. 5 reflects the outcome of the PCA described in section 2.2, illustrating the variance explained by each PC. The middle part is a heatmap displaying the effect of each PC (PC1 to PC10) on disease risk in a CoxPH model that adjusts for age, sex, BMI, alcohol intake, smoking status, and type 2 diabetes at the time of the input MRI. A white square indicates no significant association (P > 0.05); A dotted square without a number signifies a significant association (P < 0.05) that did not persist after adjusting for multiple comparisons (PBH-adjusted > 0.05). A dotted square with a number indicates a significant association (P < 0.05) that remained significant after multiple comparison adjustments (PBH-adjusted < 0.05). Hazard ratios (HR) are converted to percent change in risk for easier interpretation. The bottom part of the figure is a breakdown of the top 5 radiomic features contributing to selected PCs, i.e., PC2, PC6, and PC10.

### 3. Technical implementation details, FIG. 1

Various aspects of the present disclosure are described by narrative text, flowcharts, block diagrams of computer systems and/or block diagrams of the machine logic included in computer program product (CPP) embodiments. With respect to any flowcharts, depending upon the technology involved, the operations can be performed in a different order than what is shown in a given flowchart. For example, again depending upon the technology involved, two operations shown in successive flowchart blocks may be performed in reverse order, as a single integrated step, concurrently, or in a manner at least partially overlapping in time.

CPP embodiment is a term used in the present disclosure to describe any set of one, or more, storage media (also called mediums) collectively included in a set of one, or more, storage devices that collectively include machine readable code corresponding to instructions and/or data for performing computer operations specified in a claim. A storage device is any tangible device that can retain and store instructions for use by a computer processor. Without limitation, the computer readable storage medium may be an electronic storage medium, a magnetic storage medium, an optical storage medium, an electromagnetic storage medium, a semiconductor storage medium, a mechanical storage medium, or any suitable combination of the foregoing. Some known types of storage devices that include these mediums include: hard disk, random access memory (RAM), read-only memory (ROM), erasable programmable read-only memory (EPROM or Flash memory), static random-access memory (SRAM), compact disc read-only memory (CD-ROM), digital versatile disk (DVD), memory stick, floppy disk, mechanically encoded device (such as punch cards or pits / lands formed in a major surface of a disc) or any suitable combination of the foregoing.

A computer readable storage medium is not to be construed as storage in the form of transitory signals per se, such as radio waves or other freely propagating electromagnetic waves, electromagnetic waves propagating through a waveguide, light pulses passing through a fibre optic cable, electrical signals communicated through a wire, and/or other transmission media. As will be understood by the skilled person, data is typically moved, on occasions, during normal operations of a storage device, such as during access, de-fragmentation, or garbage collection, but this does not render the storage device as transitory because the data is not transitory while it is stored.

Computing environment 100 is an example of an environment for the execution of at least some of the computer code involved in performing the present methods, such as segmentation, feature extraction, and weighting of extracted features (see block 150) as described herein. In addition to block 150, the computing environment 100 includes, for example, computer 101, wide area network (WAN) 102, end user device (EUD) 103, remote server 104, public cloud 105, and private cloud 106. In this embodiment, computer 101 includes processor set 110 (including processing circuitry 120 and cache 121), communication fabric 111, volatile memory 112, persistent storage 113 (including operating system 122 and block 150, as identified above), peripheral device set 114 (including user interface (UI) device set 123, storage 124, and peripheral storage 125), and network module 115. Remote server 104 includes remote database 130. Public cloud 105 includes gateway 140, cloud orchestration module 141, host physical machine set 142, virtual machine set 143, and container set 144.

COMPUTER 101 may take the form of a desktop computer, laptop computer, tablet computer, smart phone, smart watch or other wearable computer, mainframe computer, or any other form of computer or mobile device now known or to be developed in the future that is capable of running a program, accessing a network, or querying a database, such as remote database 130. Computer 101 is assumed to include one or several interface units that allows it to receive 3D images. As is well understood in the art of computer technology, and depending upon the technology, performance of a computer-implemented method may be distributed among multiple computers and/or between multiple locations. That said, the following discussion is focused on a single computer, specifically computer 101, in the interest of simplicity. Computer 101 may possibly be in a cloud.

PROCESSOR SET 110 includes one, or more, computer processors of any type now known or to be developed in the future. Processing circuitry 120 may be distributed over multiple packages, for example, multiple, coordinated integrated circuit chips. Processing circuitry 120 may implement multiple processor threads and/or multiple processor cores. Cache 121 is memory that is located in the processor chip package(s) and is typically used for data or code that should be available for rapid access by the threads or cores running on processor set 110. Cache memories are typically organized into multiple levels depending upon relative proximity to the processing circuitry. Alternatively, some, or all, of the cache for the processor set may be located off chip.

Computer readable program instructions are typically loaded onto computer 101 to cause a series of operational steps to be performed by processor set 110 of computer 101 and thereby effect a computer-implemented method, such that the instructions thus executed will instantiate the methods specified in flowcharts and/or narrative descriptions of computer-implemented methods included in this document (collectively referred to as the inventive methods). These computer readable program instructions are stored in various types of computer readable storage media, such as cache 121 and the other storage media discussed below. The program instructions, and associated data, are accessed by processor set 110 to control and direct performance of the inventive methods. In computing environment 100, at least some of the instructions for performing the inventive methods may be stored in block 150 in persistent storage 113.

COMMUNICATION FABRIC 111 is the signal conduction path that allows the various components of computer 101 to communicate with each other. Typically, this fabric is made of switches and electrically conductive paths, such as the switches and electrically conductive paths that make up buses, bridges, physical input / output ports and the like. Other types of signal communication paths may be used, such as fibre optic communication paths and/or wireless communication paths.

VOLATILE MEMORY 112 is any type of volatile memory now known or to be developed in the future. Examples include dynamic type random access memory (RAM) or static type RAM. Typically, volatile memory 112 is characterized by random access, but this is not required. In computer 101, the volatile memory 112 is located in a single package and is internal to computer 101, but, alternatively or additionally, the volatile memory may be distributed over multiple packages and/or located externally with respect to computer 101.

PERSISTENT STORAGE 113 is any form of non-volatile storage for computers that is now known or to be developed in the future. The non-volatility of this storage means that the stored data is maintained regardless of whether power is being supplied to computer 101 and/or directly to persistent storage 113. Persistent storage 113 may be a read only memory (ROM), but typically at least a portion of the persistent storage allows writing of data, deletion of data and re-writing of data. Some familiar forms of persistent storage include magnetic disks and solid-state storage devices. Operating system 122 may take several forms, such as various known proprietary operating systems or open-source Portable Operating System Interface-type operating systems that employ a kernel. The code included in block 150 typically includes at least some of the computer code involved in performing the inventive methods.

PERIPHERAL DEVICE SET 114 includes the set of peripheral devices of computer 101. Data communication connections between the peripheral devices and the other components of computer 101 may be implemented in various ways, such as Bluetooth connections, Near-Field Communication (NFC) connections, connections made by cables (such as universal serial bus (USB) type cables), insertion-type connections (for example, secure digital (SD) card), connections made through local area communication networks and even connections made through wide area networks such as the internet. In various embodiments, UI device set 123 may include components such as a display screen, speaker, microphone, wearable devices (such as goggles and smart watches), keyboard, mouse, printer, touchpad, game controllers, and haptic devices. Storage 124 is external storage, such as an external hard drive, or insertable storage, such as an SD card. Storage 124 may be persistent and/or volatile. In embodiments where computer 101 is required to have a large amount of storage (for example, where computer 101 locally stores and manages a large set of 3D images) then this storage may be provided by peripheral storage devices 125 designed for storing very large amounts of data, such as a storage area network (SAN) that is shared by multiple, geographically distributed computers.

NETWORK MODULE 115 is a collection of computer software, hardware, and firmware, which allows computer 101 to communicate with other computers through WAN 102. Network module 115 may include hardware, such as modems or Wi-Fi signal transceivers, software for packetizing and/or de-packetizing data for communication network transmission, and/or web browser software for communicating data over the internet. In some embodiments, network control functions and network forwarding functions of network module 115 are performed on the same physical hardware device. In other embodiments (for example, embodiments that utilize software-defined networking (SDN)), the control functions and the forwarding functions of network module 115 are performed on physically separate devices, such that the control functions manage several different network hardware devices. Computer readable program instructions for performing the inventive methods can typically be downloaded to computer 101 from an external computer or external storage device through a network adapter card or network interface included in network module 115.

WAN 102 is any wide area network (for example, the internet) capable of communicating computer data over non-local distances by any technology for communicating computer data, now known or to be developed in the future. In some embodiments, the WAN 102 may be replaced and/or supplemented by local area networks (LANs) designed to communicate data between devices located in a local area, such as a Wi-Fi network. The WAN and/or LANs typically include computer hardware such as copper transmission cables, optical transmission fibres, wireless transmission, routers, firewalls, switches, gateway computers and edge servers.

END USER DEVICE (EUD) 103 is any computer system that is used and controlled by an end user (for example, a customer of an enterprise that operates computer 101) and may take any of the forms discussed above in connection with computer 101. EUD 103 typically receives helpful and useful data from the operations of computer 101. For example, in a hypothetical case where computer 101 is designed to provide a recommendation to an end user, this recommendation would typically be communicated from network module 115 of computer 101 through WAN 102 to EUD 103. In this way, EUD 103 can display, or otherwise present, the recommendation to an end user. In some embodiments, EUD 103 may be a client device, such as thin client, heavy client, mainframe computer, desktop computer and so on.

REMOTE SERVER 104 is any computer system that serves at least some data and/or functionality to computer 101. Remote server 104 may be controlled and used by the same entity that operates computer 101. Remote server 104 represents the machine(s) that collect and store helpful and useful data for use by other computers, such as computer 101. For example, in a hypothetical case where computer 101 is designed and programmed to provide a recommendation based on historical data, then this historical data may be provided to computer 101 from remote database 130 of remote server 104.

PUBLIC CLOUD 105 is any computer system available for use by multiple entities that provides on-demand availability of computer system resources and/or other computer capabilities, especially data storage (cloud storage) and computing power, without direct active management by the user. Cloud computing typically leverages sharing of resources to achieve coherence and economies of scale. The direct and active management of the computing resources of public cloud 105 is performed by the computer hardware and/or software of cloud orchestration module 141. The computing resources provided by public cloud 105 are typically implemented by virtual computing environments that run on various computers making up the computers of host physical machine set 142, which is the universe of physical computers in and/or available to public cloud 105. The virtual computing environments (VCEs) typically take the form of virtual machines from virtual machine set 143 and/or containers from container set 144. It is understood that these VCEs may be stored as images and may be transferred among and between the various physical machine hosts, either as images or after instantiation of the VCE. Cloud orchestration module 141 manages the transfer and storage of images, deploys new instantiations of VCEs and manages active instantiations of VCE deployments. Gateway 140 is the collection of computer software, hardware, and firmware that allows public cloud 105 to communicate through WAN 102.

Some further explanation of virtualized computing environments (VCEs) will now be provided. VCEs can be stored as images. A new active instance of the VCE can be instantiated from the image. Two familiar types of VCEs are virtual machines and containers. A container is a VCE that uses operating-system-level virtualization. This refers to an operating system feature in which the kernel allows the existence of multiple isolated user-space instances, called containers. These isolated user-space instances typically behave as real computers from the point of view of programs running in them. A computer program running on an ordinary operating system can utilize all resources of that computer, such as connected devices, files and folders, network shares, CPU power, and quantifiable hardware capabilities. However, programs running inside a container can only use the contents of the container and devices assigned to the container, a feature which is known as containerization.

PRIVATE CLOUD 106 is similar to public cloud 105, except that the computing resources are only available for use by a single enterprise or organization. While private cloud 106 is depicted as being in communication with WAN 102, in other embodiments a private cloud may be disconnected from the internet entirely and only accessible through a local/private network. A hybrid cloud is a composition of multiple clouds of different types (for example, private, community or public cloud types), often respectively implemented by different vendors. Each of the multiple clouds remains a separate and discrete entity, but the larger hybrid cloud architecture is bound together by standardized or proprietary technology that enables orchestration, management, and/or data/application portability between the multiple constituent clouds. In this embodiment, public cloud 105 and private cloud 106 are both part of a larger hybrid cloud.

While the present invention has been described with reference to a limited number of embodiments, variants, and the accompanying drawings, it will be understood by those skilled in the art that various changes may be made, and equivalents may be substituted without departing from the scope of the present invention. In particular, a feature (device-like or method-like) recited in a given embodiment, variant or shown in a drawing may be combined with or replace another feature in another embodiment, variant or drawing, without departing from the scope of the present invention. Various combinations of the features described in respect of any of the above embodiments or variants may accordingly be contemplated, that remain within the scope of the appended claims. In addition, many minor modifications may be made to adapt a particular situation or material to the teachings of the present invention without departing from its scope. Therefore, it is intended that the present invention is not limited to the particular embodiments disclosed, but that the present invention will include all embodiments falling within the scope of the appended claims. In addition, many other variants than explicitly touched above can be contemplated. For example, other segmentation and feature extraction algorithms can be contemplated.

## Claims

1. A computer-implemented method for determining risks of having or developing medical conditions, and/or complications thereof, in a subject, the method comprising:
accessing a 3D image (41) of a skeletal muscle of the trunk of the subject, wherein the 3D image has been previously obtained using a medical imaging technique,
segmenting (S43 - S44) the 3D image (41) using an automatic image segmentation process;
extracting (S45) muscle radiomics features from the segmented 3D image (44); and
based on predetermined sets of weights assigned to respective endpoints, executing (S50) a module to determine a score for each of the endpoints by weighting the extracted muscle radiomics features in accordance with a respective one of the predetermined sets of weights, wherein
said score is a risk of having or developing a medical condition or a complication thereof for the respective endpoint, and
said medical condition is primarily linked to a system or an organ that is anatomically distinct from said skeletal muscle.

2. The computer-implemented method according to claim **1,** wherein at least some of the extracted muscle radiomics features include features related to:
a pattern of fat infiltration in said skeletal muscle, where said pattern preferably captures one or more textural features of said skeletal muscle, and
a shape of said skeletal muscle or a portion thereof.

3. The computer-implemented method according to claim **2,** wherein
one of the extracted muscle radiomics features further relates to a degree of fat infiltration in said skeletal muscle.

4. The computer-implemented method according to claim **3,** wherein said at least some of the extracted muscle radiomics features include features related to:
a content of fat infiltration in said skeletal muscle, which preferably is a muscle at the abdominal level of the subject,
the pattern of fat infiltration in the skeletal muscle, where this pattern captures several textural features of the skeletal muscle, and
a shape of said skeletal muscle or a portion thereof.

5. The computer-implemented method according to any one of claim **1** to **4,** wherein
said automatic image segmentation process causes to segment the 3D image (41) by:
obtaining (S43) a segmentation mask (50) from a first image obtained from the 3D image, and
applying (S44) the segmentation mask (50) obtained to a second image obtained from the 3D image (41) to obtain the segmented 3D image (44), wherein the first image and the second image contain different information in terms of fat content and/or water content, and,
preferably, the 3D image (41) is an MRI image, the first image (43) is a water map, and the second image (42) is a fat fraction map, whereby the segmented 3D image (44) is obtained as a segmented fat fraction map.

6. The computer-implemented method according to claim **5,** wherein
the segmentation mask is obtained (S43) by running a trained neural network, preferably a convolutional neural network, on the first image (43), and
said neural network is a computational model that has been trained to infer segmentation masks from a set of annotated images, which are preferably annotated water maps.

7. The computer-implemented method according to any one of claims **1** to **6,** wherein
the endpoints are associated with medical conditions that include Parkinson's disease, acute pancreatitis, and/or a renal disease, and
the endpoints are associated with medical conditions that include each of Parkinson's disease, acute pancreatitis, and the renal disease.

8. The computer-implemented method according to any one of claims **1** to **7,** wherein
the module further takes patient information and/or genetic information of the patient as input, in addition to the muscle radiomics features, and
the genetic information preferably contains single nucleotide polymorphisms, or SNPs, more preferably myosteatosis-associated SNPs.

9. The computer-implemented method according to any one of claims **1** to **8,** wherein the method further comprises:
accessing an additional 3D image (41) of an additional organ that is anatomically distinct from said skeletal muscle, wherein the additional 3D image (41) has been previously obtained using a medical imaging technique;
segmenting (S43 - S44) the additional 3D image (41) using an automatic image segmentation process; and
extracting (S45) additional radiomics features from the segmented, additional 3D image, to obtain a set of radiomics features that include both the muscle radiomics features and the additional radiomics features, whereby the module is executed (S50) to determine said score for each of the endpoints by weighting the radiomics features of said set,
and wherein, preferably,
one or more of the medical conditions associated with said endpoints are primarily related to said additional organ.

10. The computer-implemented method according to any one of claims **1** to **9,** wherein
the extracted muscle radiomics features are weighted in accordance with said respective one of the predetermined sets of weights by performing, for each of the endpoints, a dot product of two vectors, wherein a first vector of the two vectors includes the extracted muscle radiomics features and a second vector of the two vectors includes said one of the predetermined sets of weights, and
the computer-implemented method further preferably comprises, prior to performing said dot product, concatenating,
on the one hand, additional features with the extracted muscle radiomics to form said first vector, the additional features reflecting additional information, and,
on the other hand, additional weights assigned to said additional features with said one of the predetermined sets of weights.

11. The computer-implemented method according to any one of claims **1** to **10,** wherein the method further comprises performing a preliminary analysis (S10 - S30) comprising:
accessing a set of 3D images of skeletal muscles of trunks of multiple subjects, wherein the 3D images have been previously obtained using said medical imaging technique, for example magnetic resonance imaging, the set of 3D images preferably including at least 30000 3D images of respective subjects;
segmenting (S13 - S16) the 3D images using said automatic image segmentation process;
extracting (S17) sets of muscle radiomics features from the segmented 3D images;
performing (S21) a dimension reduction on all sets of extracted radiomics features to obtain linear combinations of the radiomics feature values extracted;
statistically mapping (S22) the linear combinations onto said endpoints; and
determining (S23) said sets of weights, thanks to constants of the linear combinations, and assigning the sets of weights determined to their respective endpoints.

12. The computer-implemented method according to claim **11,** wherein
the dimension reduction is performed using a principal component analysis, by keeping only the first L principal components, where L is preferably between 8 and 12, and more preferably equal to 10.

13. The computer-implemented method according to claim **11** or **12,** wherein
the linear combinations are statistically mapped (S22) onto said endpoints by running a time-to-event model, which preferably is a Cox proportional hazards time-to-event model, such that weighting the extracted muscle radiomics features results in time-dependent quantities, which are preferably expressed as relative risk measures.

14. A computerized system for determining risks of having or developing medical conditions, and/or complications thereof, in a subject, the computerized system comprising:
an interface unit configured to receive 3D images that have been previously obtained using a medical imaging technique; and
processing means (110, 112, 150), which are configured to:
access a 3D image (41) of a skeletal muscle of the trunk of the subject,
segment the 3D image (41) using an automatic image segmentation process;
extract muscle radiomics features from the segmented 3D image (44); and
based on predetermined sets of weights assigned to respective endpoints, execute a module to determine a score for each of the endpoints by weighting the extracted muscle radiomics features in accordance with a respective one of the predetermined sets of weights, wherein
said score is a risk of having or developing a medical condition or a complication thereof for the respective endpoint, and
said medical condition is primarily linked to a system or an organ that is anatomically distinct from said skeletal muscle.

15. A computer program comprising software code adapted to perform a method for determining risks of having or developing medical conditions, and/or complications thereof, in a subject, according to the method of any one of claims **1** to **13** when executed by processing means.
